# FASCICULE DE BREVET EUROPEEN

(11) **EP 2 480 273 B1**
(45) Date de publication et mention de la délivrance du brevet: **14.08.2013**
(21) Numéro de dépôt: 10757605.0
(22) Date de dépôt: 27.09.2010
(51) Int. Cl.: A61M 5/145, A61M 5/315

(54) **DISPOSITIF D'INJECTION ASSISTÉE**
GESTÜTZTE INJEKTIONSVORRICHTUNG
ASSISTED INJECTION DEVICE

(30) Priorité: 26.09.2009 EP 09171427
(43) Date de publication de la demande: 01.08.2012
(73) Titulaire: Anteis SA, 1228 Plan-Les-Ouates (CH)
(72) Inventeur: VINCHON, Cyrille, F-74270 Chilly (FR); GUIST'HAU, Vincent, F-49300 Cholet (FR)
(74) Mandataire: KATZAROV S.A.
(86) Numéro de dépôt international: PCT/EP2010/064225
(87) Numéro de publication internationale: WO 2011/036285

(56) Documents cités:
- EP-A1- 1 518 575
- WO-A1-2010/100883
- US-A- 5 080 648

## Description

La présente invention concerne un dispositif d'injection assistée. La présente invention concerne en particulier un dispositif pour assister l'injection d'un produit cosmétique et/ou thérapeutique pour des applications de médecine humaine et/ou vétérinaire.

L'augmentation du volume tissulaire, par exemple, peut être souhaité à la fois dans le cas d'applications thérapeutiques ou dans un but cosmétique. Elle peut être effectuée par introduction d'une solution viscoélastique à base de produits permanents ou biodégradables dans les tissus biologiques. Ces produits peuvent présenter des propriétés de viscoélasticité très différentes, en fonction de l'indication traitée, mais également en fonction de la technologie de formulation du produit. Il existe ainsi des produits hautement fonctionnalisés par réticulations ou greffage d'un ou plusieurs polymères, possédant une seule phase, réticulée ou non, sous forme de gel ou plusieurs phases, réticulées ou non, intégrant parfois des particules solides dans la matrice de polymère. Au sein d'une même technologie, des gammes de produits ont été développées afin de répondre au besoin des différentes indications, chacun de ces produits présentant des propriétés viscoélastiques et d'écoulement différentes. Ces différences de propriétés sont d'autant plus exacerbées que les moyens d'administration qui leur sont associés, que ce soient les aiguilles, les canules ou les corps de seringue peuvent être très différents en taille, diamètre ou longueur, ce qui renforce la diversité de l'offre au praticien.

Dans le cas d'applications thérapeutiques, ce type de solution viscoélastique est utilisé pour certains tissus qui nécessitent d'être élargis pour assurer leur fonction : il s'agit par exemple des cordes vocales, de l'oesophage, des sphincters ou de l'urètre.

Dans le cas des applications cosmétiques, ce type de solution viscoélastique est utilisé par exemple, pour le comblement des rides, le masquage des cicatrices, l'augmentation du volume des lèvres, le remodelage du contour du visage, la reconstruction morphologique ou la réjuvénation des couches superficielles de la peau.

Des techniques très différentes d'injection peuvent être utilisées en fonction de l'indication traitée. A titre d'exemple de techniques d'injection fréquemment utilisées, on peut citer les techniques d'injection en hachures, ou d'injection linéaire rétrotraçante, d'injection en éventail, d'injection antéro-traçante, de nappage, de dépôt local en quantité significative, d'injection en papules ou en micro-papules, ou d'injection point par point ou multipoints. Ces techniques se classent plus globalement en 2 catégories : les techniques d'injection continue, et les techniques d'injection point-par point. Il n'est en outre pas rare que ces différentes techniques soient combinées au cours d'une même séance de traitement d'un patient, selon les indications traitées.

Selon l'indication traitée, les tissus peuvent présenter des niveaux de résistance très différente. Outre les différences physiologiques inter-patients, les tissus du derme superficiel, du derme moyen ou profond, de l'hypoderme ou de zone intramusculaire ou des muqueuses, ainsi que les tissus profonds du périoste ou supra-périoste possèdent des caractéristiques de densité ou de laxité très différentes qui vont opposer des niveaux de résistance variable à l'écoulement des produits injectés. Chaque type de tissu considéré individuellement ne peut pas non plus être considéré comme un milieu complètement homogène ou amorphe. Ceci est d'autant plus vrai dans le cas de présence de tissus cicatriciels, faisant suite à des traumatismes antérieurs et à une reconstruction plus ou moins fibreuse de ces tissus.

L'injection de produit dans le corps d'un être humain, ou d'un animal, est un procédé délicat nécessitant un certain savoir-faire pour éviter au patient des douleurs et autres effets secondaires inutiles et/ou pour doser la quantité exacte de produit injecté.

Cela est d'autant plus vrai pour l'injection de produits à but cosmétique, en particulier pour l'injection de produits de comblement ou de réjuvénation tels que par exemple les gels réticulés ou non à base d'acide hyaluronique ou d'un de ses sels. En effet, il est particulièrement important lors de l'injection de ces produits de contrôler avec précision la quantité de matière injectée à chaque endroit afin d'obtenir l'effet cosmétique voulu et d'éviter au maximum les effets secondaires liés au traumatisme de la perforation par l'aiguille ou liés à l'expansion voire la dilacération des tissus lors de l'injection. Ces effets secondaires se traduisent par des douleurs pendant et après l'injection et/ou des rougeurs, irritations, oedème, hématomes, inflammation, induration, etc. après l'injection du produit.

Selon le niveau de sévérité de la dépression à combler dans le cas des produits de comblement, ou du niveau de desséchement de la peau associé à son type et épaisseur, dans le cas de produits de réjuvénation, ou selon la sévérité de la pathologie à traiter, dans le cas de produit avec des indications thérapeutiques, le praticien habilité sait estimer la quantité de produit nécessaire et le mode de répartition à appliquer. En revanche, la diversité dans l'offre des produits et des moyens d'administration, la variété des techniques et des profondeurs d'injection, et les différences de résistance tissulaire rendent difficile le dosage de la quantité de produit pour les praticiens.

Afin de réduire au maximum le traumatisme lié à la perforation créée par l'aiguille, les produits sont injectés au travers d'aiguilles fines, de calibre compris généralement entre 16 G et 36G, plus généralement entre 21 G et 32G. Il en résulte des forces d'éjection très importantes, qui rendent difficile la maîtrise de la localisation de l'injection (site et profondeur) et du volume injecté.

Il faut également noter que les produits viscoélastiques utilisés dans les indications cosmétique et/ou thérapeutique, afin de séparer, remplacer, supplémenter ou combler des tissus mous, ou en augmenter le volume, sont des produits sensibles à la dégradation mécanique et au cisaillement propre aux conditions extrêmes d'injection et aux variations rapides de pression induites par la résistance à l'injection, qu'elle soit liée aux tissus eux-mêmes ou aux moyens d'administration. Tous ces produits présentent des courbes de viscosité en fonction du taux de cisaillement caractérisées par une chute rapide des propriétés de viscosité.

D'autre part, il arrive que l'aiguille se détache au cours de l'injection en raison d'une pression continue trop importante ou de changements brusques de pression dans la seringue, dus par exemple à une forte résistance à l'injection des tissus environnants. Ce phénomène est d'autant plus fréquent lors de l'injection de produits à viscosité élevée, tels que par exemple certains produits destinés à des applications cosmétiques, et en particulier quand ces produits sont injectés au travers d'aiguilles fines.

Il existe des dispositifs d'injection assistée dans lesquels le produit à injecter est expulsé hors de la seringue à l'aide d'un fluide sous pression, d'un ressort ou d'un moteur exerçant une force d'injection déterminée sur le fond mobile de la seringue. De tels dispositifs ne permettent cependant pas une régulation satisfaisante du débit ou de la quantité de produit injecté car la force nécessaire à l'éjection d'une certaine quantité de produit à un certain débit dépendra de la résistance des tissus dans laquelle l'aiguille de la seringue est introduite, du choix des moyens d'administration (aiguille, seringue) et des propriétés viscoélastiques du produit injecté. La force d'injection nécessaire sera donc différente pour chaque patient, voire pour chaque site d'injection sur un même patient.

De par les propriétés de viscoélasticité et les fortes viscosités des produits injectés, ces dispositifs d'injection assistée ne permettent en outre pas de stopper l'écoulement de façon satisfaisante à la fin de la dépose, ce qui perturbe également le contrôle de la quantité injectée lors du cycle suivant. L'arrêt seul de la poussée sur le fond mobile de la seringue ne permet pas d'éviter la relaxation de l'énergie emmagasinée dans le gel et un écoulement résiduel au bout de l'aiguille. L'application d'une poussée négative (traction) sur le fond mobile de la seringue va créer, quant à elle, presque instantanément une dépression dans le gel, et l'aspiration d'un volume d'air en bout d'aiguille, ce qui perturbe également le contrôle de la quantité injectée lors du cycle suivant. Cette difficulté de stopper avec précision l'écoulement du produit injecté est d'autant plus problématique dans les cas d'application point par point où l'injection du produit est arrêtée fréquemment.

D'autres dispositifs d'injection assistée comprennent un système de poussée volumétrique, ce qui leur permet un meilleur contrôle de la quantité de produit injecté quelle que soit la résistance des tissus dans lesquels l'injection a lieu. Un tel dispositif comprend généralement un mécanisme permettant de transformer par exemple la rotation d'un moteur électrique en une avance linéaire du fond de la seringue. Ce mécanisme comprend par exemple une vis couplée à l'axe de rotation du moteur et tournant dans un écrou bloqué en rotation, provoquant ainsi la translation de l'écrou qui est par exemple lié au fond de la seringue par l'intermédiaire d'un piston rigide. Ainsi, par le contrôle précis du nombre de tours effectués par le moteur et de sa vitesse de rotation, il est possible de déterminer précisément la quantité de produit injecté et son débit, indépendamment de la pression à la sortie de l'aiguille de la seringue, du choix des moyens d'administration (aiguille, seringue) ou des propriétés viscoélastiques du produit injecté. Le document EP 1518575 décrit un dispositif d'injection correspondant au préambule de la revendication 1. Un désavantage de tels dispositifs est cependant que le lien mécanique direct entre le moteur et le fond de la seringue provoque parfois des changements de pression rapides dans la seringue contenant le produit à injecter, ce qui peut occasionner des douleurs au patient et/ou provoquer le détachement de l'aiguille, comme expliqué plus haut.

Un but de la présente invention est de proposer un dispositif pour l'injection de produits cosmétiques et/ou thérapeutiques permettant un contrôle précis de la quantité et du débit du produit injecté, tout en évitant la douleur et/ou les effets secondaires subis par les patients.

Un autre but de la présente invention est de proposer un dispositif pour l'injection assistée de produits cosmétiques et/ou thérapeutiques permettant de diminuer le risque de douleurs pour le patient et/ou de détachement de l'aiguille au cours de l'injection par rapport aux dispositif pour l'injection assistée de l'art antérieur.

Ces buts sont atteints par un dispositif comprenant les caractéristiques de la revendication indépendante.

Ces buts sont atteints en particulier par un dispositif d'injection assistée comprenant un réservoir pour contenir un produit à injecter, le réservoir comprenant un fond mobile ; une aiguille fixée sur l'extrémité du réservoir opposée au fond mobile ; un piston agissant sur le fond mobile pour expulser à travers l'aiguille un produit à injecter se trouvant dans le réservoir ; un moteur rotatif pour déplacer le piston par l'intermédiaire d'un mécanisme d'entrainement transformant le mouvement rotatif du moteur rotatif en un mouvement linéaire du piston, le piston agissant sur le fond mobile par l'intermédiaire d'une pièce d'amortissement compressible.

La présente invention sera mieux comprise à la lecture de la description qui suit illustrée par les figures, où :
- la figure 1 représente un dispositif d'injection assistée selon une forme d'exécution préférentielle de l'invention ;
- la figure 2 est une coupe partielle de la pièce à main du dispositif d'injection assistée de la figure 1 ;
- la figure 3 est un détail de la figure 2 ;
- la figure 4 illustre les effets du dispositif de l'invention sur la régulation du débit lors de l'injection.

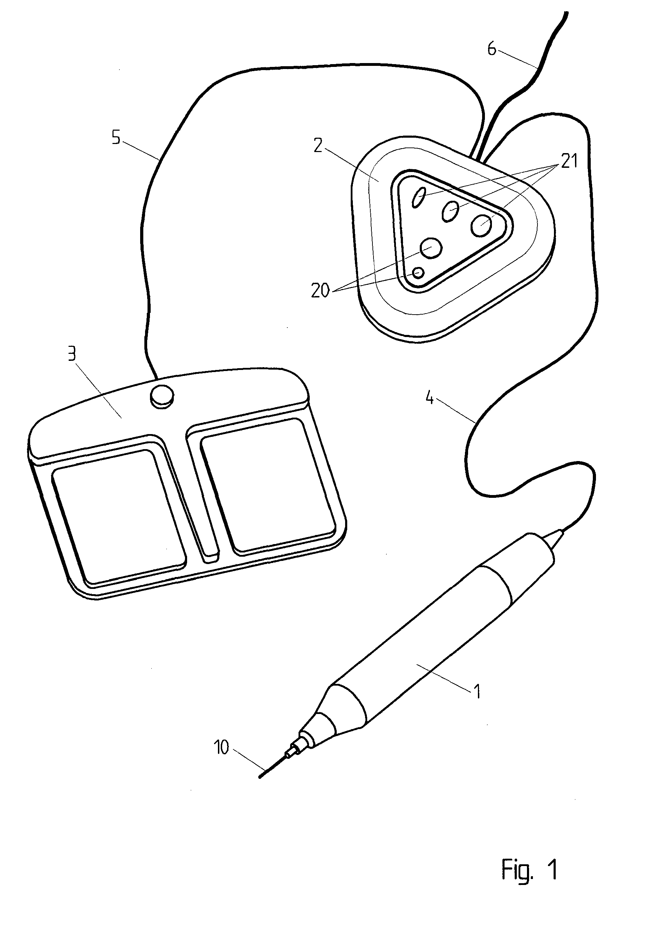

En référence à la figure 1, un dispositif d'injection assistée comprend typiquement une pièce à main 1, un boîtier de contrôle 2 et un pédalier 3.

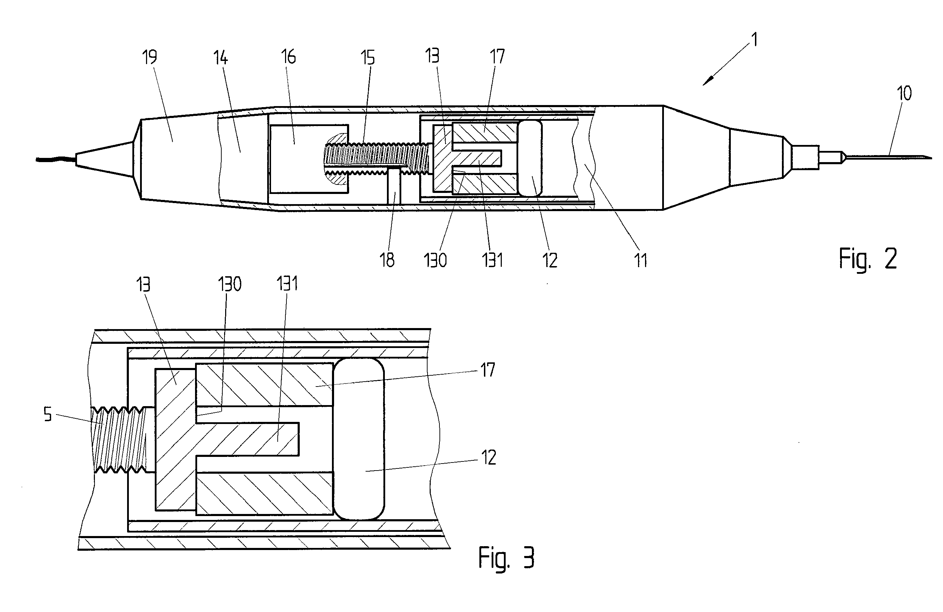

La pièce à main 1, illustrée plus en détail à la figure 2, comprend un réservoir 11 destiné à contenir un produit à injecter, sur lequel est fixée une aiguille 10 permettant l'injection du produit contenu dans le réservoir 11. De préférence, le réservoir 11 est allongé, par exemple cylindrique, et l'aiguille 10 est fixée sur une des ses deux extrémités, l'autre extrémité du réservoir étant fermée par un fond mobile 12. Selon l'invention, un piston 13 agissant sur le fond mobile 12 du réservoir est entrainé par un moteur électrique rotatif 14 au travers d'un mécanisme d'entrainement pour transformer la rotation du moteur 14 en un mouvement de translation du piston 13, permettant ainsi un contrôle précis du volume et du débit de produit injecté.

Selon une forme d'exécution préférentielle de l'invention représentée à titre d'exemple illustratif mais non limitatif à la figure 2, le mécanisme d'entrainement comprend un écrou 16 en rotation avec le moteur 14, entrainé par exemple directement par l'axe du moteur 14, et une vis 15 au moins partiellement vissée dans l'écrou 16 et bloquée en rotation relativement au moteur 14, par exemple à l'aide d'un ergot de guidage linéaire 18. L'ergot de guidage linéaire 18 est par exemple fixé à la paroi intérieure du boîtier 19 de la pièce à main 1 et au moins partiellement inséré par exemple dans une gorge pratiquée le long de la vis 15, empêchant ainsi la vis de tourner par rapport au boîtier 19, tout en permettant sa translation par rapport à ce dernier. Ainsi, lorsque le moteur 14 tourne, il entraîne l'écrou 16 qui tourne sur place et la vis 15 est déplacée en translation le long de son axe à l'intérieur de la pièce à main 1. L'ergot de guidage linéaire 18 est par exemple formé par moulage lors de la fabrication du boîtier 19.

La vis 15 est ainsi guidée en translation et bloquée en rotation à l'intérieur de la pièce à main 1 tandis que l'écrou 16 est bloqué en translation et guidé en rotation par le moteur 14. Le piston 13 agissant sur le fond mobile 12 du réservoir 11 est alors lié à la vis 15 qui l'entraine dans son mouvement de translation. Le piston 13 est de préférence fixé, par exemple soudé, à l'extrémité de la vis 15, de sorte que le piston 13 est entraîné par la vis 15 dans les deux directions le long de l'axe de la vis 15. Selon une variante, le piston 13 et la vis 15 sont formés d'une seule pièce, par exemple par tournage.

Selon une autre forme d'exécution non représentée, la vis est entraînée en rotation par l'axe du moteur et bloquée en translation, tandis que l'écrou est bloqué en rotation et libre de se déplacer en translation dans la pièce à main le long de l'axe de la vis. La rotation du moteur, et donc de la vis, provoque alors le déplacement linéaire de l'écrou à l'intérieur de la pièce à main et le piston agissant sur le fond mobile du réservoir est lié à l'écrou qui l'entraine dans son mouvement de translation. Le piston est alors de préférence fixé, par exemple soudé, à l'écrou, ou le piston et l'écrou sont formés d'une seule pièce.

Connaissant le pas de la vis 15, il est possible de déterminer exactement l'amplitude et la vitesse des déplacements du piston 13 en fonction des mouvements du moteur 14. En particulier, la direction du déplacement du piston 13 dépend du sens de rotation du moteur 14, la vitesse de déplacement du piston 13 dépend de la vitesse de rotation du moteur 14 et l'amplitude du déplacement du piston 13 dépend du nombre de tours effectués par le moteur 14.

Ainsi, par un contrôle précis du nombre de tours effectué par le moteur 14 et de son sens et de sa vitesse de rotation, la quantité de produit injectée et le débit de l'injection peuvent être déterminés et régulés avec précision, indépendamment de la pression à la sortie de l'aiguille 10, de la définition des moyens d'administration, en particulier de la taille de l'aiguille 10, ou des propriétés viscoélastiques du produit injecté.

Le pas de vis de la vis 15 est de préférence faible pour permettre un réglage précis des déplacements du piston 13 et pour minimiser l'amplitude des variations du couple du moteur 14, et donc par exemple de son alimentation en courant électrique, nécessaires pour compenser les effets des variations de la résistance à l'injection rencontrée.

D'autres formes du mécanisme d'entrainement sont toutefois possibles dans le cadre de l'invention pour transformer la rotation du moteur 14 en un déplacement linéaire du piston 13. Selon une variante, le mécanisme d'entrainement comprend par exemple une roue dentée entrainée par le moteur et agissant sur une tige crantée faisant office de piston. Selon une autre forme d'exécution, le mécanisme d'entrainement comprend par exemple deux vis parallèles, une première vis étant liée au moteur et entrainant en rotation la seconde vis qui se déplace selon un axe linéaire par rapport à la première.

La pièce à main 1 comprend de préférence un boîtier 19 en un matériau rigide et de forme ergonomique, permettant sa bonne prise en main par l'utilisateur lors de l'injection. L'essentiel des éléments de la pièce à main 1 sont de préférence logés dans le boîtier 19, à l'exception d'une partie au moins de l'aiguille 10.

De préférence, le moteur et le mécanisme d'entrainement sont logés dans la pièce à main 1. D'autres formes d'exécution sont toutefois possibles dans le cadre de l'invention. Selon une variante, le moteur est logé dans le boîtier de contrôle afin de minimiser le volume de la pièce à main, et le mécanisme d'entrainement agit sur le piston, par exemple à l'aide d'un câble en acier semi-rigide, au travers du lien souple reliant le boîtier de contrôle à la pièce à main.

En référence à la figure 1, le boîtier de contrôle 2 du dispositif d'injection comprend par exemple un microprocesseur, de la mémoire et un logiciel stocké dans la mémoire et exécutable par le microprocesseur, qui lui permettent de contrôler avec précision la quantité et le débit de produit à injecter expulsé de la pièce à main 1 à travers l'aiguille, 10 par le contrôle précis des mouvements du moteur, en particulier de son sens de rotation, de sa vitesse de rotation et du nombre de tours effectués à chaque activation. Le boîtier de contrôle 1 permet par exemple de réguler l'intensité et/ou la tension du courant électrique délivré au moteur électrique rotatif afin de garantir au moteur des mouvements contrôlés réguliers et précis, indépendamment de la résistance à l'injection rencontrée.

Le boîtier de contrôle 1 comprend de préférence des boutons de commande 20 permettant à l'utilisateur de choisir un débit et/ou une quantité de produit à injecter et/ou de choisir entre une injection continue ou goutte-à-goutte. Le boîtier comprend de préférence également des voyants lumineux 21 et/ou un affichage digital et/ou un dispositif de signalisation sonore permettant à l'utilisateur de contrôler visuellement et/ou auditivement les réglages effectués et/ou le dosage en cours. Au cours de l'injection, le logiciel mis en oeuvre dans le boîtier de contrôle 2 assure le fonctionnement régulier et précis du moteur en fonction des paramètres de débit et/ou de quantité introduits préalablement par l'utilisateur.

L'activation du moteur est de préférence commandée à l'aide d'un pédalier 3, permettant ainsi à l'utilisateur de disposer de ses deux mains pour guider l'insertion de l'aiguille 10 sous la peau du patient, et éventuellement les mouvements de l'aiguille 10 au cours de l'injection.

La pièce à main 1, le boîtier de commande 2 et le pédalier 3 sont de préférence reliés entre eux par des liens de préférence souples, par exemple des câbles de communication et/ou d'alimentation 4, 5 permettant une communication entre ces éléments, en particulier la communication de signaux de commande, de signaux de contrôle et/ou la transmission d'énergie électrique. Le dispositif d'injection assistée de l'invention est de préférence alimenté par une source d'énergie électrique extérieure non représentée, auquel il est relié par exemple à l'aide d'un câble électrique d'alimentation 6.

Selon l'invention, le piston agit sur le fond mobile du réservoir par l'intermédiaire d'une pièce d'amortissement située entre le piston et le fond mobile. Une forme d'exécution préférentielle de la pièce d'amortissement est illustrée dans les figures 2 et 3 à titre d'exemple illustratif mais non limitatif. La pièce d'amortissement 17 possède de préférence des capacités de déformation propres à absorber les variations brutales de pression dans le produit contenu dans le réservoir 11 induites par les variations de résistance à l'injection, qu'elles soient liées aux tissus eux-mêmes ou aux moyens d'administration. Elle permet également d'atténuer si nécessaire les accélérations du moteur, par exemple au début de l'injection ou lors de fortes corrections de la poussée en raison de fortes variations de la résistance à l'injection rencontrée.

Selon l'invention, en cas d'augmentation rapide de la pression à l'intérieur du réservoir 11 au cours de l'injection, la pièce d'amortissement 17 se situant entre le fond mobile 12 du réservoir 11 et le piston 13 est momentanément comprimée, ce qui ralentit momentanément le déplacement du fond mobile 12 et évite ainsi une surpression dans le réservoir 11. L'énergie emmagasinée dans la pièce d'amortissement 17 est ensuite restituée au fond mobile 12 lorsque la pression dans le réservoir 11 a de nouveau diminué, par exemple suite à la diminution de la résistance à l'injection et/ou suite à l'arrêt du moteur. La pièce d'amortissement 17 permet ainsi également de réduire le niveau de cisaillement du produit contenu dans le réservoir 11 et donc de le protéger contre une forme de dégradation.

En référence à la figure 3, la pièce d'amortissement 17 est de préférence un cylindre flexible, par exemple en élastomère. L'élastomère de la pièce d'amortissement 17 a par exemple une dureté comprise entre 20 et 100 shore A, préférentiellement entre 50 et 80 shore A.

L'extrémité du piston 13 comprend de préférence un épaulement 130 permettant au piston 13 d'appuyer sur la pièce d'amortissement 17 et optionnellement une tige centrale 131 pouvant coulisser librement à l'intérieur de la pièce d'amortissement 17. De préférence, l'épaulement 130 et la tige centrale 131 sont formés d'une seule pièce, par exemple par tournage et/ou moulage d'une pièce cylindrique en un matériau rigide, par exemple du téflon, un métal, etc.

Selon une variante de l'invention, le piston 13 possède également une certaine flexibilité et contribue ainsi également à l'amortissement des variations de pression à l'intérieur du réservoir 11. Le piston 13 est cependant de préférence plus rigide que la pièce d'amortissement 17.

Selon une forme d'exécution, la pièce d'amortissement 17 est une pièce indépendante insérée dans le réservoir et appuyant contre le fond mobile 12 du réservoir 11. Selon une autre forme d'exécution, la pièce d'amortissement 17 est fixée au fond du réservoir 11, faisant par exemple partie de la même pièce que le fond mobile 12. La pièce d'amortissement 17 et le fond mobile 12 du réservoir 11 sont alors par exemple moulés en une seule pièce d'un matériau flexible, par exemple d'élastomère.

Au cours de l'injection du produit contenu dans le réservoir 11, l'épaulement 130 du piston 13 appuie sur la face de la pièce d'amortissement 17 à l'opposé du fond mobile 12. La déformation de la pièce d'amortissement 17 sous cet appui permet d'atténuer les variations brutales de pression au niveau du produit à injecter et le protège. Si le piston 13 comprend une tige centrale 131, cette dernière permet en outre de limiter la déformation de la pièce d'amortissement 17 en appuyant directement sur le fond mobile 12 après une compression déterminée de la pièce d'amortissement 17. Cela a en particulier pour effet de limiter les éventuelles imprécisions de régulation du débit ou de la quantité de produit injecté dues à une déformation trop importante de la pièce d'amortissement 17, et/ou de limiter l'écoulement de produit involontaire après l'arrêt du moteur qui serait dû à une trop forte décompensation de la pièce d'amortissement 17.

Selon le type d'injection, le piston 13 est par exemple retiré en arrière à la fin d'une série d'injections, ce qui permet de décompenser la pièce d'amortissement 17. Cette décompensation s'accompagne du retour de la pièce d'amortissement 17 à sa forme au repos, d'une libération rapide de la pression résiduelle dans le produit et à un arrêt de tout écoulement résiduel en bout d'aiguille. Selon une forme préférentielle de l'invention, le piston 13 ne crée alors pas de phénomène de retrait du fond mobile 12 ni d'aspiration car il n'entraine pas le fond mobile 12 dans son retour, la tige centrale 131, si elle est présente, coulissant librement dans la pièce d'amortissement 17. La pièce d'amortissement 17 récupère sa forme au repos et recule jusqu'au rééquilibrage à la pression atmosphérique de chaque coté du produit (coté aiguille et coté fond mobile 12), sans aspiration d'air en bout d'aiguille.

Selon l'invention, la pièce d'amortissement 17 permet grâce à sa compressibilité une montée initiale progressive du débit de produit injecté, jusqu'à l'atteinte du débit cible, selon une accélération croissante, minimisant ainsi le traumatisme des tissus en les habituant progressivement à l'augmentation de volume.

La compressibilité de la pièce d'amortissement 17 permet également de protéger le réservoir 11 des changements brutaux de pression, minimisant ainsi les risques de décrochage de l'aiguille.

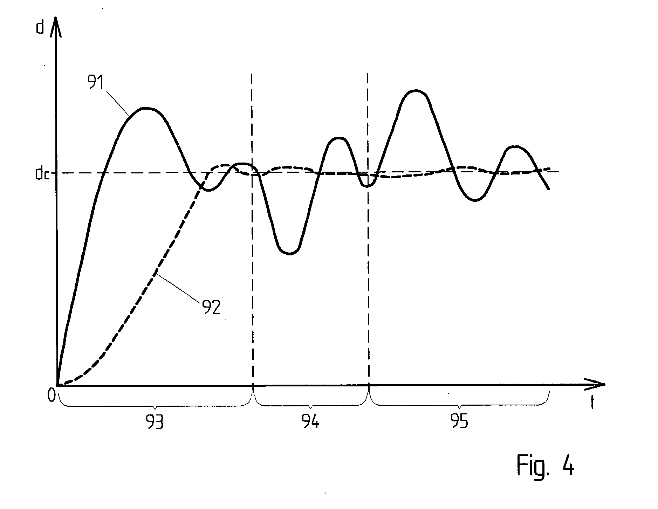

La figure 4 illustre à titre d'exemple illustratif et non limitatif la régulation du débit de produit injecté en début d'injection et la régularité du débit en cours d'injection, atteintes à l'aide du dispositif d'injection assistée de l'invention. Le graphique de la figure 4 montre ainsi la variation du débit d de produit injecté en fonction du temps t entre un débit nul en début d'injection et un débit cible de, en injection manuelle ou en injection assistée à l'aide du dispositif de l'invention.

La courbe 91 illustre les variations typiques du débit de produit injecté lors d'une injection manuelle, où le praticien essaie de réguler manuellement la vitesse de déplacement du piston d'une seringue usuelle afin d'obtenir un débit aussi régulier et proche du débit cible de que possible. Au début de l'injection, tandis que l'extrémité de l'aiguille d'injection se trouve par exemple dans un tissu non fibreux 93 du patient, le praticien appuie d'abord assez fortement sur le piston afin d'arriver rapidement à ce qui lui semble être le débit cible de, puis tente de stabiliser le débit autour de ce débit cible de, généralement après une ou deux oscillations. Si le praticien déplace par exemple l'extrémité de l'aiguille pendant l'injection, qui pénètre alors par exemple dans une zone de tissu cicatriciel 94 est généralement plus dur, le débit de produit injecté aura tendance à diminuer et le praticien devra appuyer plus fort sur le piston de la seringue pour ramener le débit proche de la valeur de débit cible de. Si l'extrémité de l'aiguille pénètre par exemple à nouveau une zone de tissu non fibreux 95, le débit augmentera avant que le praticien ne remarque le changement de résistance à l'injection et tente à nouveau de corriger le débit.

La courbe 92 illustre les variations typiques du débit d'un produit injecté à l'aide d'un dispositif d'injection assistée à contrôle volumétrique selon l'invention comprenant une pièce d'amortissement. La courbe 92 montre qu'au début de l'injection, c'est-à-dire lorsque le moteur de la pièce à main se met en marche et que le piston initie son avance linéaire, l'augmentation du débit de produit injecté est progressive en raison de la compression de la pièce d'amortissement, évitant ainsi au patient toute douleur qui serait due à un brusque changement de débit et limitant également le risque de décrochage de l'aiguille. Une fois la pièce d'amortissement comprimée, le fond mobile avance avec le piston, voire légèrement plus rapidement en raison de la décompression de la pièce d'amortissement, de sorte que le débit augmente pratiquement régulièrement jusqu'à atteindre le débit cible. La stabilisation du débit d'injection autour de la valeur de débit cible de est ensuite relativement rapide grâce au contrôle volumétrique du débit qui dépend directement des mouvements bien connus et contrôlés du moteur, et les oscillations sont de faible amplitude et au moins partiellement dues à au retour de la pièce d'amortissement dans sa position de repos, ou dans une autre position de compression stable. La courbe 92 montre qu'une fois le débit cible de atteint, le débit reste essentiellement stable quel que soit le tissu dans lequel est injecté le produit et sa résistance à l'injection.

La pièce d'amortissement est décrite selon la forme d'exécution préférentielle de l'invention sous la forme d'un cylindre en matière compressible. D'autres formes d'exécution sont cependant possible dans le cadre de l'invention. Selon une forme d'exécution alternative, la pièce d'amortissement est par exemple un ressort, par exemple un ressort métallique, placé entre le piston et le fond mobile du réservoir.

Des essais cliniques confidentiels, résumés dans l'exemple ci-dessous donné à titre illustratif et non limitatif, ont démontré que l'utilisation du dispositif d'injection assistée selon l'invention comprenant une pièce d'amortissement, par exemple en élastomère, entre le piston et le fond mobile réduit la douleur et les autres effets secondaires pour le patient en comparaison à un procédé d'injection mettant en oeuvre un dispositif de poussée manuel.

### Exemple

Il a été procédé à l'évaluation clinique du dispositif d'injection assistée de l'invention, en particulier dans le cadre de l'injection de produits de comblement et de réjuvénation. L'étude a été menée par 8 praticiens dans 7 pays.

Un but de l'étude était de fournir des preuves cliniques de la sécurité et efficacité du dispositif de l'invention dans le traitement de différentes rides faciales et dans des traitements de réjuvénation. Un autre but était d'évaluer les forces, faiblesses et facilité d'utilisation du dispositif de l'invention.

Au total, 193 patients ont été impliqués dans cette étude. 130 patients ont reçu des injections de produits Mesolis ou Mesolis+ dans le cadre d'un traitement de réjuvénation. 63 patients ont reçu des injections de produits de comblement : Esthélis Soft, Esthélis Basic et/ou Fortélis. Au total, 267 indications ont été traitées avec le dispositif de l'invention : 175 pour de la réjuvénation et 92 avec des produits de comblement.

Les patients ont été sollicités par le praticien pour participer à cette évaluation alors qu'ils venaient pour recevoir soit une injection, soit pour une réjuvénation, soit pour une injection de produit de comblement, et parfois pour une combinaison des deux traitements. Pour chaque patient ayant reçu une injection, le patient lui-même et le praticien ont dû remplir des formulaires d'évaluation et des questionnaires. Les praticiens ont effectué ces essais cliniques sous accord de confidentialité.

### Résultat pour les produits de comblement

Les indications principales ont été les sillons nasogéniens et les plis d'amertume qui représentaient respectivement 47% et 11 % de l'ensemble des indications.

Fortélis a été le premier produit de comblement utilisé avec 47% des injections, juste avant Esthélis Basic avec 45% des indications traitées. Fortélis a été utilisé principalement en mode d'injection continue, ou nappage, dans 62% des cas, avec une aiguille de calibre 27G. Le mode d'injection point-par-point, ou goutte-à-goutte, a été utilisé dans 38% des traitements avec Fortélis. Esthélis Basic et Esthélis Soft sont utilisé principalement en mode d'injection continue avec une aiguille de calibre 30G.

Le mode d'injection continue a été utilisé dans plus de 81 % des indications et le mode d'injection point-par-point dans 19%.

Parmi les effets secondaires rapportés, les principaux ont été des rougeurs et des douleurs.

Après injection, les praticiens déclarent le résultat satisfaisant à 100%.

Certains patients ont eu leurs sillons nasogéniens traités avec les deux produits, Fortélis et Esthélis Basic. Fortélis en mode d'injection continue, dans le derme profond, et Esthélis Basic injecté plus superficiellement en mode d'injection continue le long des sillons.

Parmi ces patients traités avec des produits de comblement, 63% avaient déjà reçu des injections manuelles.

La plupart d'entre eux ont dit que l'injection est indolore avec le dispositif de l'invention, mais aucun d'entre eux n'a dit qu'ils avaient ressenti plus de douleurs par rapport à une injection manuelle.

Le dispositif de l'invention a été principalement utilisé pour l'injection de Fortélis. Cela aide l'injecteur à pousser le produit dans le derme.

L'indication principale a été le traitement de sillons nasogéniens. Dans ce cas, le dispositif de l'invention a été utilisé en mode d'injection goutte à goutte à vitesse élevée et une aiguille de calibre 27G.

L'utilisation du dispositif de l'invention avec des produits de comblement permet :
- une injection avec un meilleur contrôle du débit et du volume ;
- une injection moins douloureuse pour le patient : plus rapide et plus confortable, avec moins d'effets indésirables :
- une injection facilitée du Fortélis, spécialement pour les sillons profonds ;
- un meilleur résultat grâce à une meilleure intégration du produit de comblement dans le derme.

### Résultat pour la réjuvénation

Les trois indications principales traitées étaient premièrement l'hydratation du visage, à 65%, deuxièmement des pattes d'oies, à 12%, et troisièmement le cou, à 7%. Dans ces indications, le mode d'injection point-par-point a été le mode utilisé principalement. Pour la réjuvénation, Mesolis et/ou Mesolis+ ont été utilisés principalement en mode d'injection point-par-point et à faible vitesse. Le mode d'injection point-par-point a été utilisé pour 88% des indications. Une aiguille de calibre 30G a été utilisée dans 52% des cas. 46% des injections ont été effectuées avec une aiguille de calibre 32G. Les aiguilles de calibre 32G ont été utilisées principalement en mode d'injection point-par-point à faible vitesse, pour 62,5% des cas. Les aiguilles de calibre 32G sont facilement utilisables avec le dispositif de l'invention et l'injection est moins douloureuse.

L'utilisation d'un anesthésiant avant le traitement a pu être évité et moins d'effets secondaires ont été observés.

Pour l'indication principale concernant les traitements de réjuvénation, l'hydratation du visage, une moyenne de 1,2 ml de produit a été utilisée pour l'ensemble du visage. Le mode d'injection point-par-point à faible vitesse et des aiguilles de calibre 32G ont été les paramètres les plus utilisés. Aucune procédure d'anesthésie n'a été effectuée dans 74% des cas.

Parmi les effets secondaires rapportés, les principaux étaient de légères rougeurs et douleurs.

En conclusion, l'utilisation du dispositif de l'invention pour des traitements de revitalisation permet :
- une injection avec un meilleur contrôle de la vitesse et du volume des papules ;
- une utilisation dans les deux modes, injection continue et injection point-par-point, afin de permettre des techniques de micro-papulation et de nappage ;
- un retour à la vie sociale plus rapide avec des papules qui disparaissent plus vite, grâce au volume homogène des gouttes de produits délivré ;
- une injection moins douloureuse pour le patient.

### Conclusion

Ces résultats démontrent que le dispositif de l'invention est utile dans le cadre d'injections de produits de comblement et de réjuvénation. Le traitement à l'aide du dispositif de l'invention permet un meilleur contrôle de la profondeur, du débit et du volume, et l'utilisation d'aiguilles plus fines pour l'injection de produits de réjuvénation. Par conséquent, c'est moins douloureux pour la plupart des patients ; le produit a un placement meilleur et plus homogène et présente une meilleure intégration dans le derme. Il apporte des résultats satisfaisants et très peu de conséquences négatives par rapport à une injection manuelle.

En cas de revitalisation, la procédure d'anesthésie a pu être évitée, et les seuls effets secondaires ayant été rapportés sont de légères rougeurs et une faible douleur.

Avec des produits de comblement, l'injection est facile et indolore. Le produit est distribué de manière très homogène et naturelle dans le derme, plus que lors d'une injection manuelle.

Le dispositif d'injection assistée de l'invention est compatible avec toutes les techniques d'injection et tout type de produits, particulièrement des produits de revitalisation et de comblement.

Le dispositif de l'invention est facile à manipuler, puisqu'il est tenu comme un crayon, entre deux doigts.

Les patients ont rapportés qu'ils étaient satisfaits du traitement, qu'ils avaient ressenti moins de douleur avec le dispositif de l'invention par rapport à une injection manuelle. Certains d'entre eux ont rapporté qu'ils appréciaient le bruit du dispositif qui semble procurer un effet relaxant.

Les praticiens ont apprécié l'utilisation du dispositif, car il est plus précis et il permet de réduire la fatigue musculaire. Le volume des gouttes est régulier et de très petites papules peuvent être produites. Il permet un retour à la vie sociale meilleur et plus rapide, puisque moins d'effets indésirables sont remarqués.

Le dispositif de l'invention a été utilisé avec tout type de produits, de réjuvénation et de comblement, avec un niveau de satisfaction élevé, proche de 100%.

De préférence, le réservoir 11 et/ou l'aiguille 10 du dispositif d'injection assistée de l'invention sont remplaçables et/ou jetables.

Le boîtier 19 de la pièce à main 1 est par exemple formé de deux pièces assemblées par vissage, permettant ainsi l'ouverture de la pièce à main 1 et le retrait du réservoir 11 après injection du produit à injecter qu'il contenait et son remplacement par un réservoir plein, du même ou d'un autre produit à injecter. Le réservoir vide ou partiellement vide est par exemple éliminé, ou rempli à nouveau pour une nouvelle utilisation. L'aiguille 10 est également remplaçable afin de permettre son élimination ou sa stérilisation après injection, et/ou pour permettre son remplacement par une aiguille propre et désinfectée d'un même diamètre ou d'un diamètre différent, en vue d'une nouvelle injection.

Lors du retrait du réservoir 11, la pièce d'amortissement 17 reste de préférence avec le réservoir et est par conséquent également remplacée. Selon une variante, la pièce d'amortissement est conservée lors du changement de réservoir 11 et associée au nouveau réservoir.

Le dispositif d'injection assistée de l'invention est adapté à l'injection de tout produit cosmétique et/ou thérapeutique dans le cadre de la médecine humaine et/ou vétérinaire.

Il est en particulier adapté à l'injection d'un produit cosmétique et/ou thérapeutique dans les tissus mous, intradermique, sous-cutanée, ou dans le périoste, d'un patient afin de séparer, remplacer, supplémenter ou combler ces tissus, ou en augmenter le volume, qui requiert une précision particulière de la quantité de produit injectée et de sa répartition afin d'obtenir des résultats satisfaisants. Ces applications nécessitent généralement l'injection de solutions viscoélastiques dont le dosage est grandement facilité par l'utilisation du dispositif d'injection assistée de l'invention. De plus, l'injection de solutions viscoélastiques provoque souvent des douleurs lors de brusques changements du débit d'injection, qui sont évitées grâce au dispositif d'injection assistée selon l'invention.

Le dispositif d'injection assistée de l'invention est adapté à son utilisation dans toute méthode de comblement et/ou de réjuvénation de tissus mous intradermiques, sous-cutanés ou périostiques d'un patient, comprenant l'injection dans lesdits tissus du patient d'un produit de comblement et/ou de revitalisation. Le dispositif de l'invention permet en particulier au praticien, à l'aide des boutons de commande du boîtier de commande, de choisir entre une injection continue et une injection point-par-point.

Le dispositif d'injection assistée de l'invention est donc adapté à son utilisation dans toute méthode pour séparer, remplacer, combler ou supplémenter des tissus mous intradermiques, sous-cutanés ou périostiques d'un patient, comprenant les étapes d'insertion de l'extrémité de l'aiguille 10 du dispositif de l'invention dans lesdits tissus du patient et d'activation du moteur rotatif 14 du dispositif pour déplacer le piston 13 par l'intermédiaire d'un mécanisme d'entrainement 15, 16 transformant le mouvement rotatif du moteur rotatif 14 en un mouvement linéaire du piston 13 pour expulser à travers l'aiguille 10 un produit cosmétique et/ou thérapeutique se trouvant dans le réservoir 11, les variations du débit du produit cosmétique et/ou thérapeutique à travers l'aiguille 10 étant amorties par la pièce d'amortissement 17.

Le dispositif d'injection assistée de l'invention est cependant également adapté à l'injection de produits thérapeutiques et/ou cosmétiques, y compris des produits à faibles viscosité, par exemple dans les cartilages, les articulations, les veines, la cavité intraoculaire ou dans la cavité chirurgicale extra-oculaire, pour obturer, par exemple des veines, ou pour traiter ces éléments grâce à un relargage maitrisé. Le dispositif d'injection assistée selon l'invention est ainsi par exemple adapté pour des traitements dans les domaines de la rhumatologie, ophtalmologie, phlébologie, pour l'injection de toxines par exemple dans le traitement de l'hyperhydrose, et/ou pour le traitement thérapeutique multiple résultant de l'injection d'un dispositif relargant, d'un principe actif ou d'un médicament.

Plus généralement, le dispositif d'injection assistée de l'invention est adapté à son utilisation dans toute méthode thérapeutique comprenant l'injection de toxines, d'un principe actif ou d'un médicament dans un cartilage, une articulation, une veine, une cavité intraoculaire ou dans une cavité chirurgicale extra-oculaire d'un patient. L'injection peut en outre être une injection continue ou une injection point-par-point.

Le dispositif d'injection assistée de l'invention est adapté à son utilisation dans toute méthode de traitement dans le domaine par exemple de la rhumatologie, de l'ophtalmologie, de la phlébologie, du traitement de l'hyperhydrose, ou de l'obturation d'une veine d'un patient, comprenant l'injection de toxines, d'un principe actif ou d'un médicament au patient, comprenant les étapes d'insertion de l'extrémité de l'aiguille 10 du dispositif de l'invention dans un cartilage, une articulation, une veine, une cavité intraoculaire ou dans une cavité chirurgicale extra-oculaire du patient, et l'activation du moteur rotatif 14 du dispositif pour déplacer le piston 13 par l'intermédiaire d'un mécanisme d'entrainement 15, 16 transformant le mouvement rotatif du moteur rotatif 14 en un mouvement linéaire du piston 13 pour expulser à travers l'aiguille 10 des toxines, un principe actif ou un médicament se trouvant dans le réservoir 11, les variations du débit des toxines, principe actif ou médicament à travers l'aiguille 10 étant amorties par la pièce d'amortissement 17.

## Revendications

1. Dispositif d'injection assistée comprenant :
un réservoir (11) pour contenir un produit à injecter, ledit réservoir (11) comprenant un fond mobile (12) ;
une aiguille (10) fixée sur l'extrémité dudit réservoir (11) opposée audit fond mobile (12) ;
un piston (13) agissant sur ledit fond mobile (12) pour expulser à travers ladite aiguille (10) un produit à injecter se trouvant dans ledit réservoir (11), ledit piston (13) agissant sur ledit fond mobile (12) par l'intermédiaire d'une pièce d'amortissement (17);
un moteur rotatif (14) pour déplacer ledit piston (13) par l'intermédiaire d'un mécanisme d'entrainement (15, 16) transformant le mouvement rotatif dudit moteur rotatif (14) en un mouvement linéaire dudit piston (13) ;
**caractérisé en ce que** ladite pièce d'amortissement (17) est un cylindre flexible compressible et **en ce que** ledit piston (13) comprend un épaulement (130) pour appuyer sur ladite pièce d'amortissement (17) et une tige (131) s'étendant librement à l'intérieur de ladite pièce d'amortissement (17) lorsque ledit épaulement appuie sur ladite pièce d'amortissement (17) pour limiter la déformation de ladite pièce d'amortissement (17).

2. Dispositif selon la revendication précédente, ladite pièce d'amortissement (17) étant disposée dans ledit réservoir (11) contre ledit fond mobile (12).

3. Dispositif selon l'une des revendications précédentes, ladite pièce d'amortissement (17) étant en élastomère.

4. Dispositif selon l'une des revendications précédentes, ladite pièce d'amortissement (17) ayant une dureté comprise entre 20 et 100 shore A.

5. Dispositif selon la revendication précédente, ladite pièce d'amortissement (17) ayant une dureté comprise entre 50 et 80 shore A.

6. Dispositif selon l'une des revendications précédentes, ladite pièce d'amortissement (17) étant fixée audit fond mobile (12).

7. Dispositif selon l'une des revendications précédentes, ledit épaulement (130) et ladite tige (131) étant formés d'une seule pièce.

8. Dispositif selon la revendication précédente, ledit épaulement (130) et ladite tige (131) étant formés par tournage d'une pièce cylindrique.

9. Dispositif selon l'une des revendications 7 ou 8, ledit épaulement (130) et ladite tige (131) étant formés par moulage.

10. Dispositif selon l'une des revendications précédentes, ledit épaulement (130) et ladite tige (131) étant en un matériau rigide.

11. Dispositif selon la revendication précédente, ledit épaulement (130) et ladite tige (131) étant en téflon.

12. Dispositif selon la revendication 10, ledit épaulement (130) et ladite tige (131) étant en métal.

13. Dispositif selon l'une des revendications précédentes, ledit piston (13) et ladite pièce d'amortissement (17) étant configurés de sorte que lorsque le piston (13) est retiré en arrière, il n'entraine pas le fond mobile (12) dans son retour.

14. Dispositif selon l'une des revendications précédentes, ledit réservoir (11) étant remplaçable par un autre réservoir.

15. Dispositif selon l'une des revendications précédentes, ladite aiguille (10) étant remplaçable par une autre aiguille.

## Patentansprüche

1. Gestützte Injektionsvorrichtung, mit:
einem Behälter (11) zur Aufnahme des zu injizierenden Produktes, wobei der besagte Behälter (11) einen beweglichen Boden (12) aufweist;
einer Nadel (10), die am Ende des besagten Behälters (11) gegenüber dem besagten beweglichen Boden (12) angebracht ist;
einem Kolben (13), der auf den beweglichen Boden (12) wirkt, um durch die besagte Nadel (10) ein zu injiziertes Produkt, das sich im besagten Behälter (11) befindet, auszustossen, wobei der besagte Kolben (13) auf den besagten beweglichen Boden (12) durch ein Dämpfungsstück (17) wirkt;
einem Drehmotor (14), um den besagten Kolben (13) durch einen Antriebsmechanismus (15, 16) zu bewegen und der die Drehbewegung des besagten Drehmotors (14) in eine Linearbewegung des besagten Kolbens (13) umwandelt;
**dadurch gekennzeichnet, dass** das besagte Dämpfungsstück (17) ein flexibler komprimierbarer Zylinder ist und dass der besagte Kolben (13) eine Schulter (130) aufweist, um auf das besagte Dämpfungsstück (17) zu drücken sowie eine Stange (131), die sich frei in das Innere des besagten Dämpfungsstücks (17) erstreckt, um die Verformung des besagten Dämpfungsstücks (17) einzuschränken.

2. Vorrichtung gemäss dem vorhergehenden Anspruch, wobei das besagte Dämpfungsstück (17) im Behälter (11) gegen den besagten beweglichen Boden (12) angebracht wird.

3. Vorrichtung gemäss einem der vorhergehenden Ansprüche, wobei das besagte Dämpfungsstück (17) aus Elastomere besteht.

4. Vorrichtung gemäss einem der vorhergehenden Ansprüche, wobei das besagte Dämpfungsstück (17) eine Härte zwischen 20 und 100 Shore A aufweist.

5. Vorrichtung gemäss dem vorhergehenden Anspruch, wobei das besagte Dämpfungsstück (17) eine Härte zwischen 50 und 80 Shore A aufweist.

6. Vorrichtung gemäss einem der vorhergehenden Ansprüche, wobei das besagte Dämpfungsstück (17) am besagten beweglichen Boden (12) befestigt ist.

7. Vorrichtung gemäss einem der vorhergehenden Ansprüche, wobei die besagte Schulter (130) und die besagte Stange (131) aus einem einzigen Stück geformt werden.

8. Vorrichtung gemäss dem vorhergehenden Anspruch, wobei die besagte Schulter (130) und die besagte Stange (131) durch Drechseln eines zylindrischen Stücks geformt werden.

9. Vorrichtung gemäss einem der Ansprüche 7 oder 8, wobei die besagte Schulter (130) und die besagte Stange (131) durch Giessen geformt werden.

10. Vorrichtung gemäss einem der vorhergehenden Ansprüche, wobei die besagte Schulter (130) und die besagte Stange (131) aus einem rigiden Material bestehen.

11. Vorrichtung gemäss dem vorhergehenden Anspruch, wobei die besagte Schulter (130) und die besagte Stange (131) aus Teflon bestehen.

12. Vorrichtung gemäss Anspruch 10, wobei die besagte Schulter (130) und die besagte Stange (131) aus Metall bestehen.

13. Vorrichtung gemäss einem der vorhergehenden Ansprüche, wobei der besagte Kolben (13) und das besagte Dämpfungsstück (17) so konfiguriert sind, dass wenn der Kolben (13) nach hinten gezogen wird, er den beweglichen Boden (12) während seiner Rückbewegung nicht antreibt.

14. Vorrichtung gemäss einem der vorhergehenden Ansprüche, wobei der besagte Behälter (11) durch einen anderen Behälter ersetzbar ist.

15. Vorrichtung gemäss einem der vorhergehenden Ansprüche, wobei die besagte Nadel (10) durch eine andere Nadel ersetzbar ist.

## Claims

1. Assisted injection device comprising:
a reservoir (11) for containing a product to be injected, said reservoir (11) comprising a mobile bottom (12);
a needle (10) fastened onto the extremity of said reservoir (11) opposite said mobile bottom (12);
a plunger (13) actuating said mobile bottom (12) to expel through said needle (10) a product to be injected located in said reservoir (11), said plunger (13) actuating said mobile bottom (12) through a damping element (17);
a rotating motor (14) for moving said plunger (13) through a driving mechanism (15, 16) transforming the rotation movement of said rotating motor (14) into a linear movement of said plunger (13);
**characterized in that** said damping element (17) is a flexible compressible cylinder and **in that** said plunger (13) comprises a shoulder (130) for pressing on said damping element (17) and a rod (131) extending freely inside said damping element (17) when said shoulder presses on said damping element (17) to limit the deformation of said damping element (17).

2. Device according to the preceding claim, wherein said damping element (17) is placed in said reservoir (11) against said mobile bottom (12).

3. Device according to one of the preceding claims, wherein said damping element (17) is made of elastomer.

4. Device according to one of the preceding claims, wherein said damping element (17) exhibits a hardness comprised between 20 and 100 shore A.

5. Device according to the preceding claim, wherein said damping element (17) exhibits a hardness comprised between 50 and 80 shore A.

6. Device according to one of the preceding claims, wherein said damping element (17) is fastened to said mobile bottom (12).

7. Device according to one of the preceding claims, wherein said shoulder (130) and said rod (131) are formed of a single part.

8. Device according to the preceding claim, wherein said shoulder (130) and said rod (131) are formed by machine-turning of a cylindrical part.

9. Device according to one of the claims 7 or 8, wherein said shoulder (130) and said rod (131) are formed by molding.

10. Device according to one of the preceding claims, wherein said shoulder (130) and said rod (131) are made of a rigid material.

11. Device according to the preceding claim, wherein said shoulder (130) and said rod (131) are made of Teflon.

12. Device according to claim 10, wherein said shoulder (130) and said rod (131) are made of metal.

13. Device according to one of the preceding claims, wherein said plunger (13) and said damping element (17) are configured so that when the piston (13) is drawn backwards, it does not drive the mobile bottom (12) upon its return.

14. Device according to one of the preceding claims, wherein said reservoir (11) can be replaced by another reservoir.

15. Device according to one of the preceding claims, wherein said needle (10) can be replaced by another needle.
